# EUROPEAN PATENT SPECIFICATION

(11) **EP 3 183 252 B1**
(45) Date of publication and mention of the grant of the patent: **12.05.2021**
(21) Application number: 15753374.6
(22) Date of filing: 21.08.2015
(51) Int. Cl.: C07D 487/04

(54) **OXALATE SALT OF RUXOLITINIB**
OXALATSALZ NACH RUXOLITINIB
SEL D'OXALATE DE RUXOLITINIB

(30) Priority: 21.08.2014 EP 14181843
(43) Date of publication of application: 28.06.2017
(73) Proprietor: ratiopharm GmbH, 89079 Ulm (DE)
(72) Inventor: ALBRECHT, Wolfgang, 89075 Ulm (DE); SELIG, Roland, 89077 Ulm (DE)
(74) Representative: D Young & Co LLP
(86) International application number: PCT/EP2015/069296
(87) International publication number: WO 2016/026974

(56) References cited:
- WO-A1-2007/070514
- WO-A1-2012/174061
- WO-A2-2008/157208
- STEFFEN PAULEKUHN G ET AL: "Trends in Active Pharmaceutical Ingredient Salt Selection based on Analysis of the Orange Book Database", JOURNAL OF MEDICINAL CHEMISTRY, AMERICAN CHEMICAL SOCIETY, vol. 50, no. 26, 27 December 2007 (2007-12-27), pages 6665-6672, XP002651069, ISSN: 0022-2623, DOI: 10.1021/JM701032Y [retrieved on 2007-12-01]

## Description

### FIELD OF THE INVENTION

The present invention relates to Ruxolitinib oxalate salt, to solid state forms thereof, processes for the preparation thereof and formulations thereof.

### BACKGROUND OF THE INVENTION

(R)-3-(4-(7H-pyrrolo [2,3-d] pyrimidin-4-yl)-1H-pyrazol-1-yl)-3-cyclopentylpropanenitrile has the chemical formula C₁₇H₁₈N₆ and is reported in WO2007/070514 as a selective inhibitor of protein kinases, such as the enzyme Janus Kinase in its subtypes 1 and 2 (hereinafter also referred to as "JAK1" and "JAK2") with IC₅₀ values of 3.3 nM and 2.8 nM, respectively. The compound modulates JAK1/JAK2-mediated cell's signaling, which regulates a number of cytokines and growth factors that are important for hematopoiesis and immune function and as such it has been asserted that it is useful in the therapy of various diseases, e.g. cancer. The compound is also known under the INN Ruxolitinib and apparently has the following structure (I):

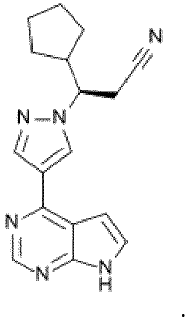

Ruxolitinib is marketed under the trade name Jakafi® in the US and Jakavi® in Europe for the treatment of patients with myelofibrosis, polycythemia vera, essential Thrombocythemia and hematological malignancies. Recently it was discussed that Ruxolitinib may also be effective for treatment of autoimmune related inflammatory diseases, such as psoriasis.

WO2007/070514 discloses the trifluoroacetate salt of Ruxolitinib as a synthetic intermediate. Pharmaceutically acceptable salts of Ruxolitinib have been reported in WO2008/157208, namely Ruxolitinib phosphate, Ruxolitinib sulfate and Ruxolitinib maleate.

A pharmaceutically acceptable salt is not only defined by its chemical composition but also by the so-called polymorphism. Polymorphism, the occurrence of different crystal forms, is a property of some molecules and molecular complexes. A single molecule, like Ruxolitinib or salts thereof, may give rise to a variety of polymorphs having distinct crystal structures and physical properties like melting point, thermal behaviors (e.g. measured by thermogravimetric analysis - "TGA", or differential scanning calorimetry - "DSC"), powder X-ray diffraction (PXRD) pattern, infrared absorption fingerprint, and solid state NMR spectrum. One or more of these techniques may be used to characterize a particular polymorph and to distinguish different polymorphic forms of a compound.

Different solid state forms (including solvated forms) of an active pharmaceutical ingredient may possess different properties. Such variations in the properties of different solid state forms and solvates may provide a basis for improving certain aspects of the API, such as its formulation, for example, by facilitating better processing or handling characteristics, changing the dissolution profile in a favorable direction, or improving stability (polymorph as well as chemical stability) and shelf-life. These variations in the properties of different solid state forms may also offer improvements to the final dosage form, for instance, if they serve to improve bioavailability. Different solid state forms and solvates of an active pharmaceutical ingredient may also give rise to a variety of polymorphs or crystalline forms, which may in turn provide additional opportunities to assess variations in the properties and characteristics of a solid active pharmaceutical ingredient.

Discovering new polymorphic forms and solvates of a pharmaceutical product can provide materials having, inter alia, desirable processing properties, such as ease of handling, ease of processing, chemical and polymorphic stability upon storage and processing, and ease of purification or are useful as advantageous intermediate crystal forms that facilitate conversion to other solid state forms (including other solvates) of said pharmaceutical product.

New polymorphic forms and solvates of a pharmaceutically useful compound can also provide an opportunity to improve the performance characteristics of a pharmaceutical product. It enlarges the repertoire of materials that a formulation scientist has available for formulation optimization, for example by providing a product with different properties, e.g., better processing or handling characteristics, improved dissolution profile, or improved shelf-life. Lastly, new polymorphic forms may be prepared with improved reliability and reproducibility compared to other forms, for example, in terms of crystallinity or polymorphic purity.

The intestinal tract obeys different pH ranges, which vary from pH 1 to 2 of the empty stomach, pH 3-5 for stomach filled with chyme, to pH 5-7.5 of the colon. A dissolution screening test regime for solid state forms of an active ingredient may be set up for example at pH 1.2, pH 4.5 and pH 6.8 in order to cover a broad range of the gastro-intestinal tract.

Solubility studies showed good solubility for Ruxolitinib phosphate at pH 1.2, but considerably less solubility at higher pH points. Ruxolitinib maleate exhibited less solubility at pH 1.2 in comparison with Ruxolitinib phosphate and in addition a distinct pH dependency at pH 4.5 and 6.8. Ruxolitinib sulfate gave a moderate solubility with less pH dependency at 37°C.

It is of favor when the solid state forms of an active ingredient show good solubility in order to be available for permeation into the body.

It is further of favor if salts of active ingredients show good solubility through all pH ranges of the gastro-intestinal tract.

Object of the present invention is to find a Ruxolitinib salt, which gives better solubility for all pH ranges compared to the state of the art Ruxolitinib salts, while the salt is still pharmaceutically acceptable.

### SUMMARY OF THE INVENTION

The present invention provides a solid state form of Ruxolitinib oxalate, processes for the preparation thereof, and pharmaceutical compositions and formulations comprising the solid state form of Ruxolitinib oxalate, and processes for the preparation of the pharmaceutical compositions and formulations.

The present invention also provides the use of said solid state form of Ruxolitinib oxalate for the manufacture of pharmaceutical compositions and formulations. Accordingly, the present invention further provides a pharmaceutical composition comprising said solid state form of Ruxolitinib oxalate of the present invention. The pharmaceutical composition may additionally comprise at least one pharmaceutically acceptable excipient to form a pharmaceutical formulation that can, for example, be administered to patients in need of such treatment.

The present invention comprises a process for preparing the above-mentioned pharmaceutical formulations. The process comprises combining the solid state form of Ruxolitinib oxalate with at least one pharmaceutically acceptable excipient.

The solid state form as defined herein as well as the pharmaceutical compositions and formulations of Ruxolitinib oxalate can be used as medicaments, particularly for the treatment of cancer, myelofibrosis, polycythemia vera, essential Thrombocythemia and hematological malignancies. The present invention also provides a method of treating cancer, myelofibrosis, polycythemia vera, essential Thrombocythemia hematological malignancies and psoriasis comprising administering a therapeutically effective amount of the solid state form of Ruxolitinib oxalate of the present invention, or a therapeutically effective amount of at least one of the pharmaceutical compositions or formulations of the present invention comprising said solid state form of Ruxolitinib oxalate of the present invention to a patient in need thereof.

The present invention also provides the use of said solid state form of Ruxolitinib and/or Ruxolitinib salt, particularly Ruxolitinib oxalate, or at least one of the above pharmaceutical compositions and/or formulations for the manufacture of a medicament for treating cancer, myelofibrosis, polycythemia vera, essential Thrombocythemia, hematological malignancies and psoriasis.

### BRIEF DESCRIPTION OF THE FIGURES

Figure 1 shows a comparison of solubility of Ruxolitinib oxalate, Ruxolitinib sulfate, Ruxolitinib maleate and Ruxolitinib phosphate at pH 1.2, pH 4.5 and pH 6.8
Figure 2a shows a powder X-ray diffraction pattern of Ruxolitinib oxalate.
Figure 2b shows a ¹³C NMR spectrum of Ruxolitinib oxalate.
Figure 2c shows a ¹H NMR spectrum of Ruxolitinib oxalate.
Figure 2d shows a DSC thermogram of Ruxolitinib oxalate.
Figure 2e shows an IR spectra of Ruxolitinib oxalate
Figure 3a shows a DVS experiment with Ruxolitinib oxalate.
Figure 3b shows a comparison of powder X-ray diffraction patterns of Ruxolitinib oxalate before and after the DVS experiment.
Figure 3c shows a comparison of powder X-ray diffraction patterns of Ruxolitinib oxalate before and after the 12 week stability experiment at 40°C and 75% humidity.

### DETAILED DESCRIPTION OF THE INVENTION

The present invention provides a solid state form of Ruxolitinib oxalate, processes for preparing the solid state form, as well as pharmaceutical compositions and formulations comprising said solid state form.

In accordance with WO 2008/157208, Ruxolitinib sulfate, phosphate and maleate have high crystallinity and give good handling properties. However, WO 2008/157208 is silent for solubility over all pH ranges in the gastro-intestinal tract.

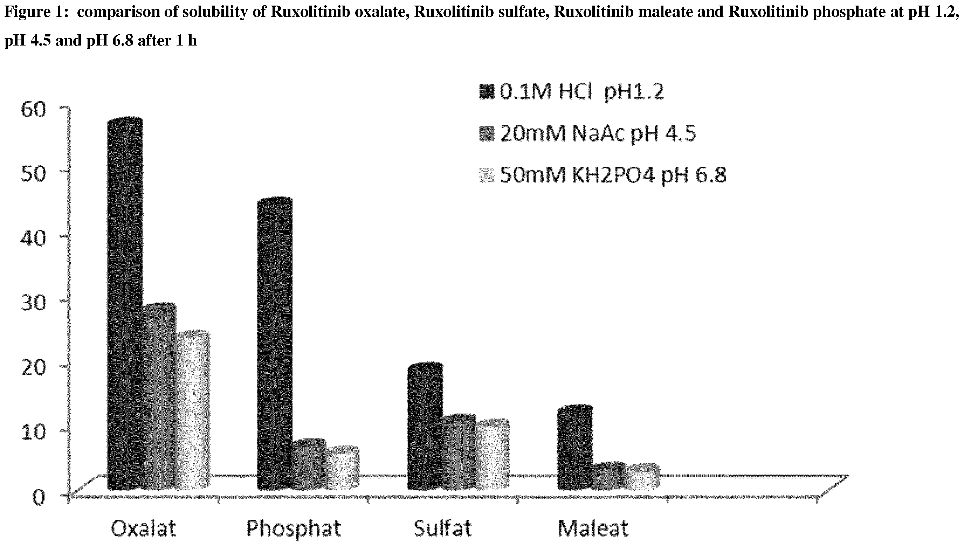

Ruxolitinib sulfate does not show such a distinct pH dependency as the phosphate salt (Figure 1), but has only moderate solubility.

Ruxolitinib phosphate is the best soluble salt known so far, but solubility decreases with rising pH. The ratio of the solubility at pH 1.2 to pH 4.5 is 7 and the ratio of pH 1.2 to pH 6.8 is 9. A similar tendency can be observed for Ruxolitinib maleate, but solubility is lower at all pH points, measured with respect to Ruxolitinib sulfate and phosphate. The ratio of the solubility at pH 1.2 to pH 4.5 is 4 and the ratio of pH 1.2 to pH 6.8 is 4 as well.

The present invention offers crystalline Ruxolitinib oxalate with improved solubility (Figure 1). Solubility at pH 1.2 is factor 1.3 better in the light of Ruxolitinib phosphate, factor 5 better at pH 4.5 and pH 6.8. However, ratio solubility of pH 1.2:4.5, as well as pH 1.2:6.8 is 2 for both ratios for Ruxolitinib oxalate, so a considerably better ratio than for Ruxolitinib phosphate in regard to the pH dependent solubility profile as well as the overall solubilities.

Further, depending on which other solid state form it is compared with, the Ruxolitinib oxalate form of the present invention may have advantageous properties selected from at least one of: chemical or polymorphic purity, increased crystallinity, flowability, solubility, dissolution rate, bioavailability, morphology or crystal habit, specific surface and pycnometric density, bulk/tap density, stability - such as chemical stability as well as thermal and mechanical stability with respect to polymorphic conversion, stability towards dehydration and/or storage stability, a lower degree of hygroscopicity, low content of residual solvents and advantageous processing and handling characteristics such as compressibility, and bulk density.

Solid state forms of Ruxolitinib oxalate comprise crystal forms, or crystalline forms, or amorphous forms of Ruxolitinib oxalate. As used herein, solid state forms, crystal forms, crystalline forms, polymorphs and polymorphic forms are used interchangeably.

A crystal form may be referred to herein as being characterized by graphical data "substantially as depicted" in a Figure. Such data include, for example, powder X-ray diffractograms, infrared spectra and DSC thermograms. The graphical data potentially provides additional technical information to further define the respective solid state form which can not necessarily or easily be described by reference to numerical values for peak positions and/or relative intensities. In any event, the skilled person will understand that such graphical representations of data may be subject to small variations, e.g., in peak relative intensities and peak positions due to factors such as variations in instrument response and variations in sample concentration and purity, which are well known to the skilled person. Nonetheless, the skilled person would readily be capable of comparing the graphical data in the Figures herein with graphical data generated for an unknown solid state form and confirm whether the two sets of graphical data are characterizing the same solid state form or two different solid state forms.

A solid state form (or polymorph) may be referred to herein as substantially free of any other crystalline (or polymorphic) forms. As used herein in this context, the expression "substantially free of any other forms" will be understood to mean that the solid state form contains 20% or less, 10% or less, 5% or less, 2% or less, or 1% or less of any other forms of the subject compound as measured, for example, by PXRD. Thus, solid state forms of Ruxolitinib oxalate described herein as substantially free of any other polymorphic forms would be understood to contain greater than 80% (w/w), greater than 90% (w/w), greater than 95% (w/w), greater than 98% (w/w), or greater than 99% (w/w) of the subject solid state form of Ruxolitinib oxalate. Accordingly, in some embodiments of the invention, the described solid state forms of Ruxolitinib oxalate may contain from 1% to 20% (w/w), from 5% to 20% (w/w), or from 5% to 10% (w/w) of one or more other solid state forms of Ruxolitinib or salts thereof.

As used herein, the expression "room temperature" or "RT" refers to a temperature between about 20 °C and about 30 °C. Usually, room temperature ranges from about 20°C to about 25 °C.

As used herein, the term "overnight" refers to a period of between about 15 and about 20 hours, typically between about 16 to about 20 hours.

As used herein, the term "reduced pressure" refers to a pressure of about 10 mbar to about 50 mbar.

As used herein, the term "isolated" corresponds to product or solid state form thereof that is physically separated from the reaction mixture in which it is formed.

As used herein, unless stated otherwise, XRPD peaks reported herein are preferably measured using CuK radiation, λ = 1.5418 Å.

As used herein, the expression "dry crystalline form" refers to a polymorph that was dried using any conventional techniques to remove residual solvent. Such conventional techniques include, but are not limited to, evaporation, vacuum drying, oven drying, drying under nitrogen flow, etc.

As used herein, and unless stated otherwise, the term "anhydrous" in relation to crystalline Ruxolitinib oxalate relates to a crystalline Ruxolitinib oxalate which contains no more than 1% (w/w) of either water or organic solvents as measured by conventional methods, for example TGA, GC or KF. An anhydrous form of the solid states of Ruxolitinib oxalate of the present invention refers to a form that does not contain crystalline water (or other solvents) in a defined, stoichiometric amount within the crystal.

Preferably, the Ruxolitinib oxalate is a Ruxolitinib mono oxalate.

As used herein, and unless indicated otherwise, the term "polymorphic stability" in relation to the crystalline forms of Ruxolitinib oxalate means that there is less than 20%, 10%, 5%, 1%, 0.5% or 0.1% (w/w) conversion of crystalline Ruxolitinib oxalate to any other solid state form of Ruxolitinib oxalate under the specified conditions.

The present invention encompasses a crystalline form of Ruxolitinib oxalate.

Preferably, the crystalline Ruxolitinib oxalate is a crystalline Ruxolitinib mono oxalate.

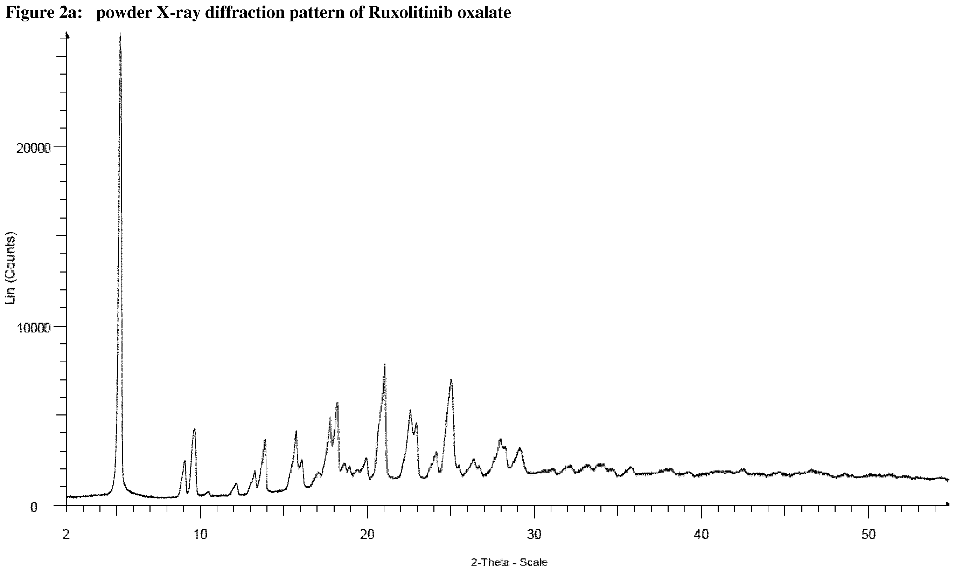

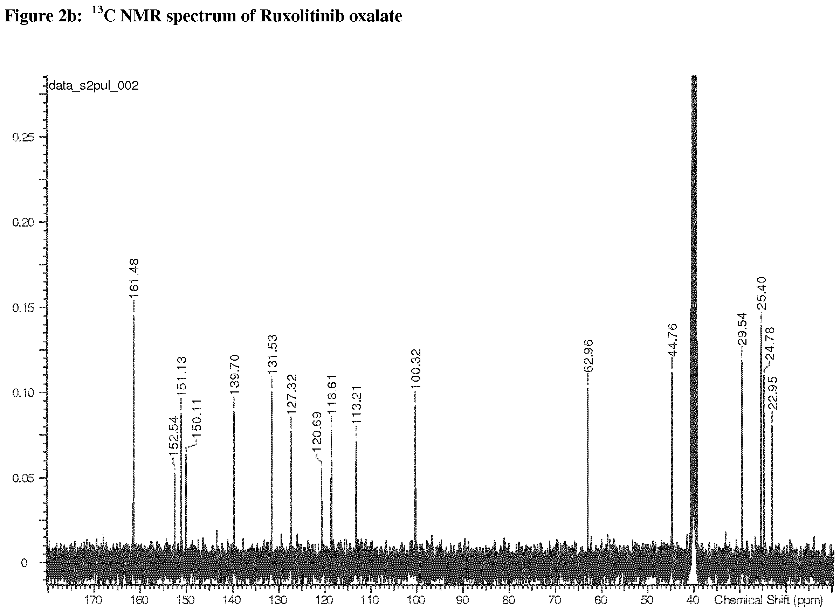

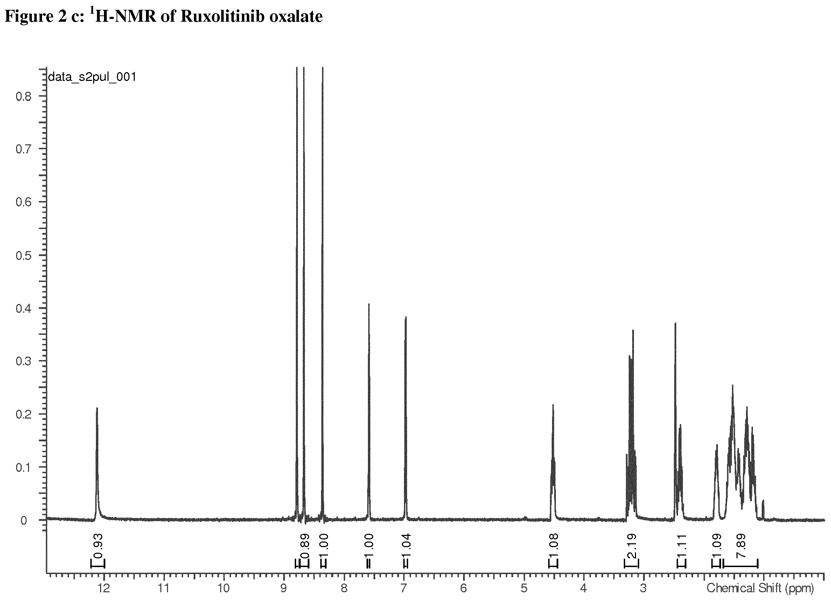

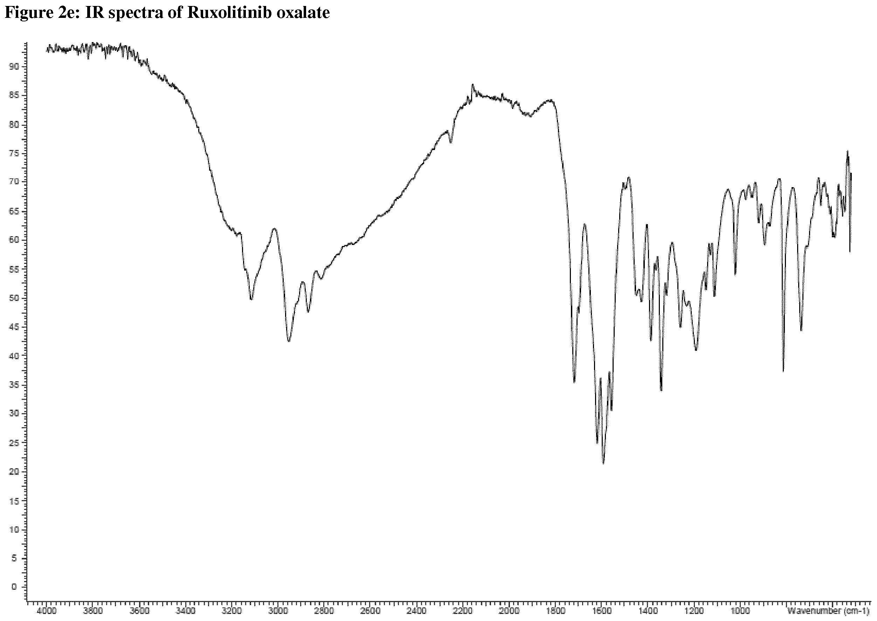

Ruxolitinib oxalate can be characterized by data selected from one or more of the following: a powder X-ray diffraction pattern having peaks at 5.2, 18.2, 21.0, 22.5 and 25.0 degrees two theta ± 0.2 degrees two theta; it may be further characterized by the X-ray powder diffraction pattern having peaks at 5.2, 18.2, 21.0, 22.5 and 25.0 degrees two theta ± 0.2 degrees two theta and also having one, two, three, four, five or six additional peaks selected from 9.0, 9.6, 13.8, 15.7, 17.7 and 22.9 degrees two theta ± 0.2 degrees two theta; and combinations of these data; a powder X-ray diffraction pattern substantially as depicted in Figure 2a.

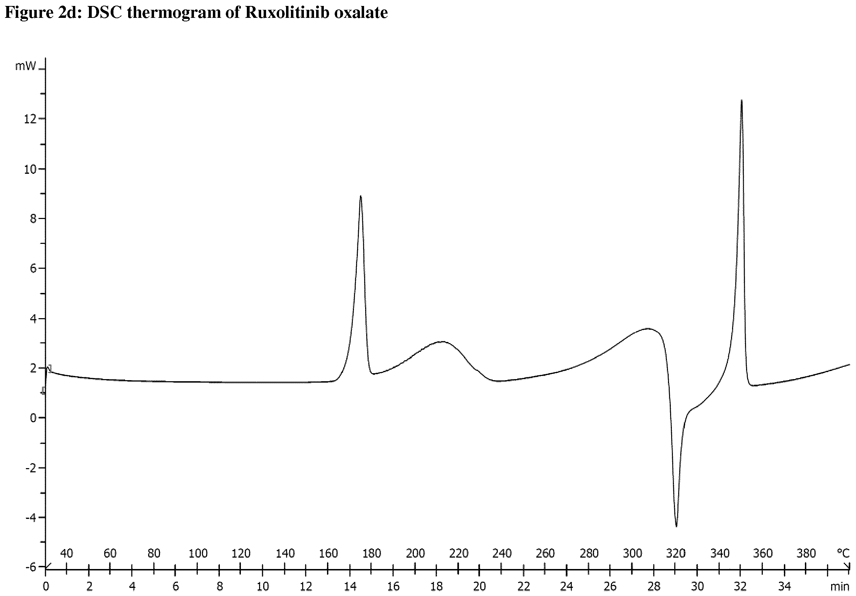

Ruxolitinib oxalate can be further characterized by one or more of the following: a DSC thermogram substantially as depicted in Figure 2d; by two sharp endotherms with onset temperatures at 170.2 ± 2°C and 346.4 ± 2°C and one sharp exotherm with onset temperature of 317.6 ± 2°C. as well as two broad signals at 186.5 ± 2°C and 265.0 ± 2°C, respectively.

Ruxolitinib oxalate can be characterized by any combinations of the above data. For example, by a powder X-ray diffraction pattern having peaks at degrees 5.2, 18.2, 21.0, 22.5 and 25.0 two theta ± 0.2 degrees two theta and also by a DSC thermogram substantially as depicted in Figure 2d.

The above Ruxolitinib oxalate has advantageous properties selected from at least one of: chemical or polymorphic purity, flowability, solubility, dissolution rate, bioavailability, morphology or crystal habit, stability - such as such as chemical stability as well as thermal and mechanical stability with respect to polymorphic conversion, storage stability, stability to dehydration, low hygroscopicity, and low content of residual solvents and advantageous processing and handling characteristics such as compressibility, or bulk density.

Particularly, crystalline Ruxolitinib oxalate has high chemical purity and excellent stability properties. Specifically, it is stable upon storage at 25°C and 60% relative humidity (RH); and at 40°C/75% RH for up to at least 12 weeks. Furthermore, crystalline Ruxolitinib oxalate has good solubility and it can be used to prepare an oral formulation, i.e. a tablet or a capsule.

The described solid state form Ruxolitinib oxalate can be used to prepare Ruxolitinib base or other different salts of Ruxolitinib, as well as solid state forms thereof and/or pharmaceutical formulations comprising one or more of the salts and/or solid state forms thereof.

The present invention further encompasses 1) a pharmaceutical composition comprising said solid state form described herein; 2) a pharmaceutical formulation comprising said solid state forms or pharmaceutical compositions described herein, and at least one pharmaceutically acceptable excipient; 3) a process to prepare such formulations comprising combining the above-described solid state forms and at least one pharmaceutically acceptable excipient; 4) the use of the above-described solid state form in the manufacture of a pharmaceutical composition, and 5) a method of treating cancer comprising administering a therapeutically effective amount of the above-described solid state forms, optionally in the form of pharmaceutical compositions or formulations. The present invention also provides a crystalline form of Ruxolitinib oxalate as described above for use as a medicament, preferably for the treatment of cancer. The pharmaceutical compositions can also be used for preparing said medicament.

Having described the invention with reference to certain preferred embodiments, other embodiments will become apparent to one skilled in the art from consideration of the specification. The invention is further defined by reference to the following examples describing in detail the preparation of the composition and methods of use of the invention. It will be apparent to those skilled in the art that many modifications, both to materials and methods, may be practiced without departing from the scope of the invention.

### ANALYTICAL METHODS

### NMR Spectroscopy

Instrument: Varian Mercury 400 Plus NMR Spectrometer, Oxford AS, 400 MHz. Samples were measured in DMSO-*d₆* at room temperature

### IR spectroscopy

### Instrument: Thermo Nicolet, Avatar 330 FT-IR with ATR.

### Differential Scanning Calorimetry (DSC)

| | |
|---|---|
| Instrument: | Mettler Toledo DSC 822E coupled with a Mettler Toledo Gas-Flow-Controller TS0800GC1 (Mettler-Toledo GmbH, Gießen, Germany) |
| Aluminium crucible: | 40 µL |
| Lid: | perforated |
| Temperature range: | 30°C to 350°C |
| Heating rate: | 10°C/ min |
| Nitrogen flush: | 50 mL / min |
| Software: | STARe Version. 8.10 |
| Interpretation: | Endothermic modus |

### X-Ray Powder Diffraction (PXRD)

The sample was analyzed on a D8 Advance X-ray powder diffractometer (Bruker-AXS, Karlsruhe, Germany). The sample holder was rotated in a plane parallel to its surface at 20 rpm during the measurement. Further conditions for the measurements are summarized in the table below. The raw data were analyzed with the program EVA (Bruker-AXS, Germany). The samples were layered onto a silicon specimen holder

| | standard measurement |
|---|---|
| radiation | Cu K_{α} (λ = 1.5418 Å) |
| source | 38 kV / 40 mA |
| detector | Vantec |
| detector slit | variable |
| divergence slit | v6 |
| antiscattering slit | v6 |
| 2θ range / ° | 2 ≤ 2θ ≤ 55 |
| step size / ° | 0.017 |

### Hygroscopicity

Vapour sorption experiments were performed in the instrument SPSx-1µ (Projekt Messtechnik, Ulm, Germany) at temperature of 25°C and the humidity cycles as shown below.

| Cycle no. | rel. humidity (% RH) | | Number of steps | Time (h) |
|---|---|---|---|---|
| | start value | end value | | |
| 1 | 40 | 0 | 4 | |
| 2 | 5 | 65 | 6 | |
| 3 | 75 | 75 | 1 | 24 h |
| 4 | 85 | 95 | 1 | |
| 5 | 90 | 0 | 9 | |
| 6 | 5 | 35 | 3 | |

### EXAMPLES

### Example 1: Preparation of Ruxolitinib Base.

Ruxolitinib base was prepared in four steps. The route of synthesis is given in the following scheme. The final three steps are described in detail.

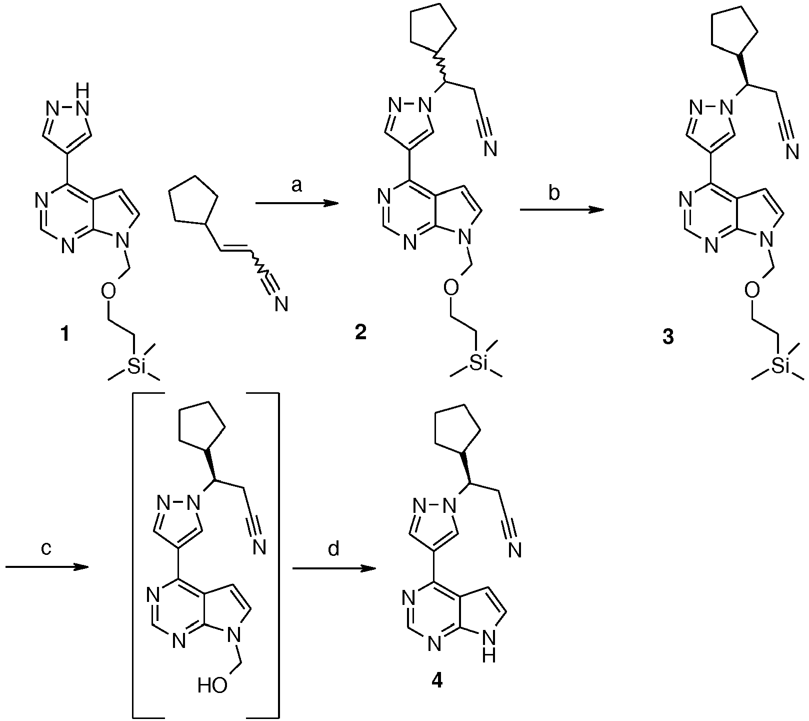

### Intermediate 2 (rac-3-Cyclopentyl-3-{4-[7-(2-trimethylsilanyl-ethoxymethyl)-7H-pyrrolo[2,3-d]pyrimidin-4-yl]-pyrazol-l-yl}-propionitrile)

231.67 g (1.90 mol) 3-Cyclopentyl-acrylonitrile and 23.70 ml (0.16 mol) 1,8-Diazabicyclo[5.4.0]undec-7-en (DBU) were added to a suspension of the starting material (1) (500 g, 1.56 mol) in 4000 ml acetonitrile (MeCN) at room temperature under stirring and nitrogen atmosphere. The reaction mixture was heated to 60°C for -22 hours, then to 80°C for 4 hours. The reaction mixture was cooled to room temperature. The cooled reaction mixture was evaporated under reduced pressure at 42°C to yield a viscous black oil. The oil was dissolved in 1300 ml dichloromethane (DCM) and 650 g silica (63-200 µm) added. The suspension was evaporated under reduced pressure at 44°C. The obtained crude product on silica was dry loaded on a silica column (1.25 kg (2.5 1) silica 63-200 µm, diameter of column 7 cm length 92 cm). The whole separation was accomplished via flash system (companion "combi flash") with a solvent flow of 100 ml/min. (pressure 5-15 bar). Elution was accomplished with EtOAc (ethylacetate)/n-hexane 1/2. The solvent was evaporated at 50°C to yield the product as highly viscous yellow oil.

### Intermediate 3 ((R)-3-Cyclopentyl-3-{4-[7-(2-trimethylsilanyl-ethoxymethyl)-7H-pyrrolo[2,3-d]pyrimidin-4-yl]-pyrazol-1-yl}-propionitrile)

The racemic starting material (1159 g; 2. 7 mol) was dissolved in 2.000 ml MeCN at 50°C. The obtained solution was transferred into a 25 1 reactor. 9560 ml of MeCN, 1960 ml THF and 1960 ml acetone were added, resulting in a yellow solution. The heating jacket was adjusted to 55°C (Ti = 51°C). (+)-Dibenzoyl tartaric acid monohydrate 2137 g (2.14 eq) was added resulting in a slightly orange solution. After -30 minutes, the solution became turbid and the heating jacket was adjusted to 80°C, after 25 minutes a clear orange solution resulted and the heating jacket was adjusted to 55°C. At 56 °C (Ti) enantiomeric pure crystals were added to initiate the crystallization and the heating jacket was adjusted to 50°C. After 1.5 h heating was turned off and the mixture was allowed to cool to room temperature and stirred overnight (Ti after 1 h 45 min. 37°C, final temperature overnight: 22°C). The precipitate was filtered off and washed with 2x100 ml of a mixture of MeCN : THF: acetone (5.8:1:1) yielding 1197 g of the tartrate. (85 % of theory, 52.4% ee).

This and another batch with an enantiomeric purity of 52.0% ee were combined.

2000 g of the combined batches were charged into a reactor and 14.48 1 of a mixture of MeCN, THF and acetone (10:1.69:1.69) were added. The reactor was heated to 80°C (Tj) (Ti = 71°C), the product was almost completely dissolved. Further 200 ml of the above solvent mixture were added, resulting in a clear solution. The reactor was cooled to 52°C (cooling rate: after 1.5 h 52°C). A colorless suspension was formed after -1.5 h. After -3.5 h the heating was turned off and the suspension was stirred overnight. (Ti final = 20°C). The product was filtered off and washed with 2 x 600 ml of the above described solvent mixture. The obtained product showed an enantiomeric purity of 80.2% ee and a yield of 1528 g (76% of theory).

1903 g of crystallized material with an optical purity of 80% ee were charged in a reactor and 13.38 1 of a mixture of MeCN/THF and acetone (10:1.69:1.69) were added. The mixture was heated to 80°C (Tj) (Ti=72.7°C), after addition of further 200 ml of the above solvent mixture, the suspension became a clear solution. The temperature was adjusted to 55°C and after 2.5 h at 52.8°C a colorless suspension was formed, the heating was turned off and stirring was continued overnight. The precipitated product was filtered off (T = 21°C) (1356 g, 92.0% ee).

1356 g of this material were charged in a reactor and 11.99 1 of a mixture of MeCN/THF and acetone (10:1.69:1.69) were added. The mixture was heated to 80°C (Tj) (Ti = 73.0°C) (clear solution). The temperature was adjusted to 55°C and after 2.5 h at 52.8°C, a colorless suspension was formed. The temperature was adjusted to 50°C. After 2 hours, heating was turned off and stirring was continued overnight. The product was filtered off (Ti = 20.3°C) and washed with 2 x 600 ml MeCN/THF/acetone (10:1.69:1.69) to yield 1063 g of the product with an enantiomeric purity of 96.6% ee.

1063 g of this material were charged in a reactor and 9390 ml MeCN/THF/acetone (10:1.69:1.69) were added. The mixture was heated to 80°C (Tj) (Ti=73.0°C) (clear solution). The temperature was adjusted to 55°C and after 1 h at 52.8°C a colorless suspension was formed. The temperature was adjusted to 50°C. After 2 hours, heating was turned off and stirring was continued overnight. The product was filtered off (Ti=20.3°C) and washed with 2 x 600 ml MeCN/THF/acetone (10:1.69:1.69) to yield 813 g of the product with an enantiomeric purity of 98.6% ee.

### Procedure to liberate the free base:

To 100 g of the tartaric acid salt were added 2000 ml ethylacetate (white suspension) and afterwards 600 ml water. The mixture was cooled to 0°C and 2N NaOH was added. The water layer was extracted with 2x 400 ml ethylacetate. The organic layers were combined and washed with 0.01M NaOH (700 ml). The organic layer was evaporated to yield 54.5 g of the product as yellow oil. Chemical purity: 99.45 %; HPLC/UV (λ= 266 nm)).

### Intermediate 4 ((R)-3-Cyclopentyl-3-[4-(7H-pyrrolo[2,3-d]pyrimidin-4-yl)-pyrazol-1-yl]-propionitrile)

The starting material (244 g; 0.559 mol) was dissolved in 2370 ml MeCN, followed by the addition of 210 ml water and lithium tetrafluoroborate (LiBF₄, 520.48 g; 5.55 mol) at room temperature (rt). The reaction temperature decreased by addition of water to 14°C (Ti= 22°C) and increased by addition of LiBF₄ to 27°C. The reaction mixture was heated to reflux (∼80°C) for 11 h. The reaction was monitored by HPLC. After complete consumption of the starting material the reaction mixture was cooled to 5°C, followed by dropwise addition of 20% aqueous ammonium hydroxide (274 ml) to adjust to pH 9. The ice bath was removed and the reaction mixture was gradually warmed to rt. After complete deprotection, which was monitored by HPLC/TLC, the reaction mixture was filtered and the solids were washed with MeCN (530 ml). The combined filtrates were evaporated under reduced pressure at 46°C and to the residue 3160 ml EtOAc and 1580 ml half saturated brine were added. The two layers were separated and the aqueous layer was extracted with EtOAc (1050 ml). The combined organic layers were washed with half saturated NaHCO₃ (1600 ml) and brine (1600 ml), dried over Na₂SO₄ and concentrated under reduced pressure to yield the crude product as orange oil. The crude product was purified by flash chromatography on silica (eluent: EtOAc). Purification procedure: the crude product was dissolved in 250 ml ethylacetate and was divided in two nearly equal portions; each portion was purified separately by a silica column (prepacked puriflash column/puriflash/1600 g/50 µm) on a Armen spot flash system flow: 80 ml/min. The obtained colorless oil was evaporated, resulting in a foam, which was triturated with 200 ml n-pentane (each portion) resulting in a colorless solid which was filtrated off and dried under vacuum at rt. The obtained product was stored under argon atmosphere in the fridge.

### Example 2: Preparation of Ruxolitinib oxalate.

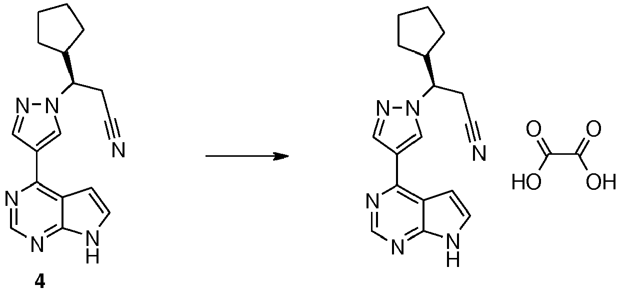

To a solution of Ruxolitinib free base (3 g; 9.8 mmol) in isopropanol (42 ml) at 60°C was added oxalic acid (0.97 g; 10.8 mmol) in isopropanol (8.4 ml). Immediately after addition the product starts to precipitate as white voluminous solid. Further 20 ml of isopropanol were added and the reaction mixture was further stirred (at 60°C) for 1.5 hours. Then the reaction mixture was cooled to room temperature and stirring was continued overnight. The precipitated product was filtrated off and dried in a vacuum oven at 50°C for 3 days to yield 3.38 g (87% of theory) of the product as colourless crystalline solid.

### Alternatively:

100 g (327.2 mmol) Ruxolitinib base (batch no. RS352) was dissolved in 2 1 isopropanol at 56°C (in flask temperature). 20.62 g (229.1 mmol, 0.7 equiv.) oxalic acid was dissolved separately in 280 ml isopropanol. After complete dissolution of Ruxolitinib, the oxalic acid was dropped into this solution at 56°C within 45 minutes. The solution became slightly turbid and after addition of approx. 100 ml (-30 minutes), a white thick suspension was formed. After complete addition of oxalic acid the reaction mixture was stirred for further 60 minutes at this temperature. The heating source was turned off and the flask was cooled to rt (flask was left in oil bath and was cooled to rt with cooling oil bath). The product was filtered off,washed with 250 ml isopropanol and dried at 50°C under vacuum.
Yield: 67.8 g (52.3 %).
¹H NMR (400 MHz, DMSO-d₆) δ ppm: 1.12 - 1.37 (m, 3 H) 1.38 - 1.64 (m, 4 H) 1.69 - 1.89 (m, 1 H) 2.35 - 2.47 (m, 1 H) 3.12 - 3.29 (m, 2 H) 4.52 (m, 1 H) 6.88 - 7.07 (m, 1 H) 6.95 - 7.00 (m, 1 H) 7.56 - 7.61 (m, 1 H) 8.35 (s, 1 H) 8.67 (s, 1 H) 8.78 (s, 1 H) 12.10 (br. s., 1 H)
¹³C NMR (400 MHz, DMSO-d₆) δ ppm: 23.0, 24.8, 25.4, 29.5, 44.8, 63.0, 100.3, 113.2, 118.6, 120.7, 127.3, 131.5, 139.7, 150.1, 151.1, 152.5, 161.5
IR (ATR) [cm⁻¹] ±2 cm⁻¹: 3115, 2953, 2870, 2816, 2253, 1718, 1618, 1591, 1556, 1450, 1439, 1427, 1387, 1342, 1259, 1192, 1113, 1022, 895, 814, 737, 590
HPLC/UV at λ= and 230 nm: > 99.9 %

### Example 3: Dynamic vapor sorption experiment with Ruxolitinib oxalate

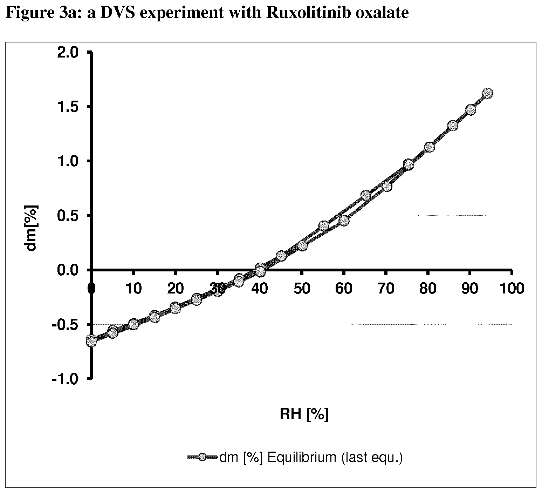

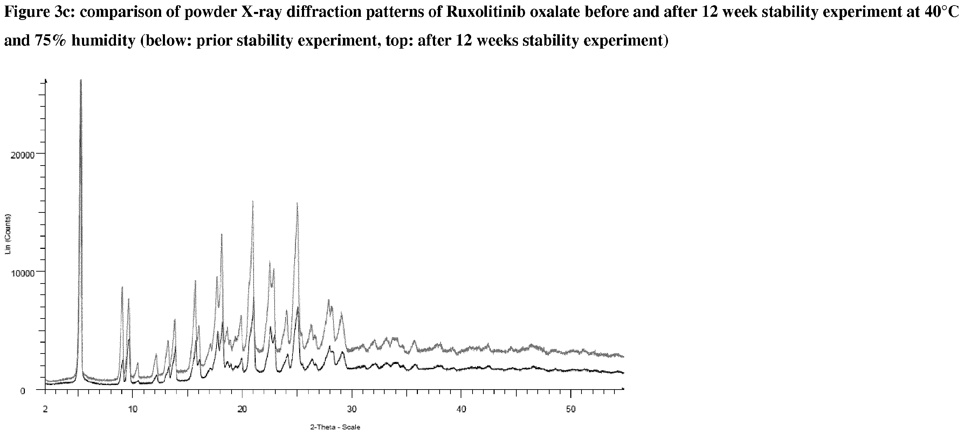

A dynamic vapor sorption experiment was performed with Ruxolitinib oxalate. At a constant temperature of 25°C, the substance was exposed to two cycles of varying relative humidity (rh) (40% → 0% →95% (75% r.h. kept constant for 48h) → 0% →40%). Figure 3a presents the relative weight change as a function of time and relative humidity and in Figure 3a the weight changes are illustrated as a function of humidity.

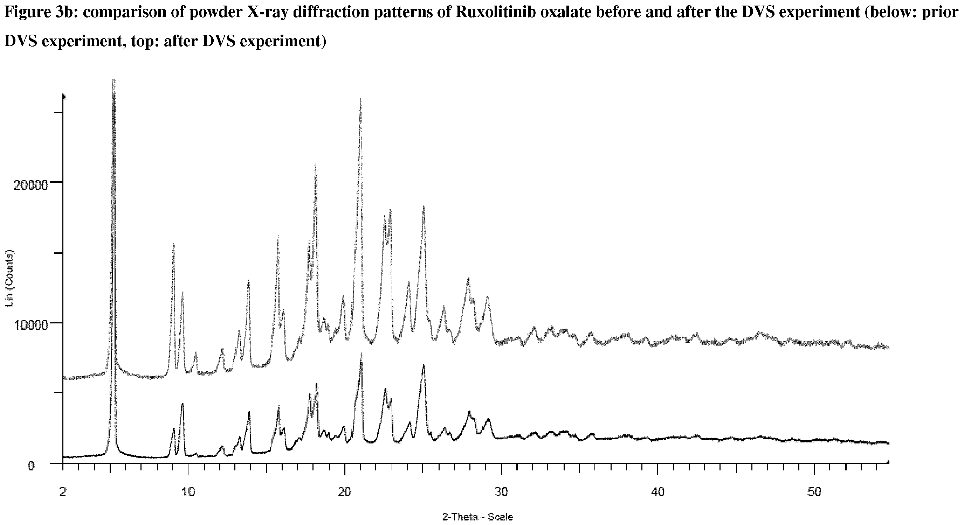

For Ruxolitinib oxalate, a relative weight change of 1% (by the end of the 75% rh step) and 1.6% (by the end of the 95% rh step) was observed. The adsorption and desorption of water was directly related to humidity which suggest that water was reversibly bound on the surface of the sample. This assumption is supported by the result of XRPD analysis of the sample, performed after the DVS experiment. The diffractogram overlay given in Figure 3b, demonstrates that the solid state of Ruxolitinib oxalate was identical to that prior to the DVS experiment.

| Time [h] | RH Set [%] | Relative weight change (dm [%]) |
|---|---|---|
| 0,00 | 40,0 | 0.0 |
| 16.7 | 0,0 | -0.60 |
| 27.7 | 45 | +0.13 |
| 95.1 | 75 | +0.96 |
| 125.3 | 95 | +1.62 |
| 180.7 | 40 | -0.02 |
| 218.1 | 0 | -0.66 |
| 227.1 | 35 | -0.11 |

### Table 1 relative weight change as a function of time and relative humidity

### Example 4: Stability.

Samples were stored in closed and open containers at 25°C/60% rh and at 40°C/75% rh and analyzed after 4, 8 and 12 weeks.

The results of determination of the chemical purity during storage at 40°C/75% rh are summarized. Both salts remained chemically stable when stored under these conditions. Therefore, the analysis of samples, which were stored at 25°C/60% rh, was omitted.

| | | Chemical purity ([area-%] at λ=230 nm) | | | |
|---|---|---|---|---|---|
| Compound | Conditions | t = 00 (= initial) | 4 weeks | 8 weeks | 12 weeks |
| Ruxolitinib phosphate | closed | 99,05 | 99,04% | 99,20% | 99,03% |
| | open | | 99,13% | 99,28% | 99,10% |
| Ruxolitinib oxalate | closed | 99,89 | 100,00% | 99,95% | 99,90% |
| | open | | 100,00% | 99,91% | 99,85% |

The solid-state stability was investigated by XRPD analysis. The results of evaluation are summarized. Ruxolitinib phosphate and Ruxolitinib oxalate remained unchanged throughout the observation period.

| | | | Evaluation of XRPD analysis | | |
|---|---|---|---|---|---|
| Compound | Conditions | | 4 weeks | 8 weeks | 12 weeks |
| Ruxolitinib phosphate | 25°C/ | closed | unchanged | unchanged | unchanged |
| | 60% rh | open | unchanged | unchanged | unchanged |
| | 40°C/ 75% rh | closed | unchanged | unchanged | unchanged |
| | | open | unchanged | unchanged | unchanged |
| Ruxolitinib oxalate | 25°C/ 60% rh | closed | unchanged | unchanged | unchanged |
| | | open | unchanged | unchanged | unchanged |
| | 40°C/ 75% rh | closed | unchanged | unchanged | unchanged |
| | | open | unchanged | unchanged | unchanged |

### Example 5: pH-dependencies of solubility (mg/ml) at 37°C

| solvent | phosphate | | | oxalate | | | sulfate | | | maleate | | |
|---|---|---|---|---|---|---|---|---|---|---|---|---|
| | 0.5 h | 1 h | 24 h | 0.5 h | 1h | 24 h | 0.5 h | 1 h | 24 h | 0.5 h | 1 h | 24 h |
| 0.1M HCl pH1.2 | N/D | 43.2 | 44.0 | 57.8 | 56.3 | 57.4 | 18.5 | 18.5 | 16.7 | 12.5 | 12.0 | 12.2 |
| 20mM sodium acetate (NaOAc/ NaAc) pH 4.5 | N/D | 6.0 | 6.8 | 24.1 | 27.7 | 29.9 | 10.4 | 10.6 | 9.5 | 2.8 | 3.2 | 2.4 |
| 50mM KH₂PO₄ pH 6.8 | N/D | 4.7 | 5.7 | 21.5 | 23.5 | 23.9 | 9.6 | 9.8 | 8.3 | 2.9 | 2.9 | 2.5 |

### Example 6 - Formulations

### Direct compression

Tablets were compressed by means of a single punch press (Korsch EKO) with a punch diameter of 8 mm, round.

The following tablets were manufactured by direct compression.

| **API + Excipient** | **[mg / DF]** | **[% / DF]** |
|---|---|---|
| Ruxolitinib oxalate | 6,50 | 4,06 |
| Prosolv SMCC | 65,02 | 40,63 |
| Carboxymethylstarch-Na | 6,00 | 3,75 |
| Povidon 25 | 4,50 | 2,81 |
| Hyprolose, Klucel EF | 7,00 | 4,37 |
| Lactose-mono, Flowlac | 70,00 | 43,75 |
| Mg stearate | 1,00 | 0,62 |

### Wet granulation

The following tablets were manufactured wet granulation.

| **API + Excipient** | **[mg / DF]** | **[% / DF]** |
|---|---|---|
| Ruxolitinib oxalate | 6,66 | 4,16 |
| Prosolv SMCC | 59,00 | 36,84 |
| Carboxymethylstarch-Na | 12,00 | 7,49 |
| Povidon 25 | 4,50 | 2,81 |
| Hyprolose, Klucel EF | 7,00 | 4,37 |
| Lactose-mono | 70,00 | 43,71 |
| Magnesium stearate | 1,00 | 0,62 |

### Process

• API, Prosolv, Lactose and Hyprolose were mixed in a high shear mixer for 15 minutes
• Wet granulation was performed with a solution of Povidon in water
• Mixture was sieved over 1 mm sieve
• Powder was dried at 40°C for 1,5 hours
• Carboxymethyl starch and Magnesium stearate were added and mixed for another 5 minutes
• Compression was performed by means of an excenter press, Korsch EKO
• Compression force 6kN
• Tablet hardness 57 N
• Tablet diameter 8 mm
• Target weight: 160.16 mg

| **API + Excipient** | **[mg / DF]** | **[% / DF]** |
|---|---|---|
| Ruxolitinib oxalate | 6,50 | 4,06 |
| Prosolv SMCC | 59,00 | 36,88 |
| Carboxymethylstarch-Na | 12,00 | 7,50 |
| Povidon 25 | 4,50 | 2,81 |
| Hyprolose, Klucel EF | 7,00 | 4,38 |
| Granulac, Lactose | 70,00 | 43,75 |
| Magnesium stearate | 1,00 | 0,63 |

### Process

- Ruxolitinib oxalate was milled in a ball mill for 15 minutes at 250 rpm and sieved 250 µm
- Milled API, Prosolv SMCC, Hyprolose and Granulac were mixed in a high shear mixer for 15 minutes
- Povidon was dissolved in water in order to get the granulation liquid
- Blended powder was granulated with granulation liquid and sieved over 1 mm sieve
- Granules were dried in a drying chamber at 40°C for 1 hour
- Carboxymethyly starch and Magnesium stearate were added and mixed for another 5 minutes
- Tablets were compressed on a single punch press Korsch, EKO
- Tablet diameter: 8 mm
- Compression force: 7 kN
- Tablet hardness: 47 N
- Target weight: 160.0 mg

## Claims

1. Salt of Ruxolitinib with oxalic acid.

2. Ruxolitinib salt of claim 1, **characterized by** a powder X-ray diffraction pattern having peaks at 5.2, 18.2, 21.0, 22.5 and 25.0 ± 0.2 degrees two theta.

3. The Ruxolitinib salt of any of the preceding claims 1-2 , further **characterized by** an additional one, two, three, four, five, or six PXRD peaks selected from 9.0, 9.6, 13.8, 15.7, 17.7 and 22.9 degrees two theta ± 0.2 degrees two theta.

4. The Ruxolitinib salt of any of the preceding claims 1-3, further **characterized by** one or more of the following: by two sharp endotherms with onset temperatures at 170.2 ± 2°C and 346.4 ± 2°C, one sharp exotherm with onset temperature of 317.6 ± 2°C, as well as two broad signals at 186.5 ± 2°C °C and 265.0 ± 2°C, respectfully.

5. The Ruxolitinib salt of any of the preceding claims 1-4, wherein the salt form is a mono oxalate

6. A pharmaceutical composition comprising the Ruxolitinib oxalate according to any of the preceding claims 1-5.

7. A pharmaceutical formulation comprising Ruxolitinib oxalate according to any of the preceding claims 1-5, and at least one pharmaceutically acceptable excipient.

8. Use of Ruxolitinib oxalate according to any of the preceding claims 1-5 in the manufacture of a pharmaceutical composition or formulation.

9. A process for preparing the pharmaceutical formulation according to claim 7, comprising combining Ruxolitinib oxalate according to any of the preceding claims 1-5, with at least one pharmaceutically acceptable excipient.

10. Ruxolitinib oxalate according to any of the preceding claims 1-5, the pharmaceutical composition according to claim 6, or the pharmaceutical formulation according to claim 7 for use as a medicament.

11. Ruxolitinib oxalate according to any of the preceding claims 1-5, the pharmaceutical composition according to claim 6, or the pharmaceutical formulation according to claim 7 for use in treating a subject suffering from cancer, myelofibrosis, polycythemia vera, essential Thrombocythemia, hematological malignancies and psoriasis.

12. Use of Ruxolitinib oxalate according to any of the preceding claims 1-5 for the preparation of: Ruxolitinib, other Ruxolitinib salts, solid state forms of Ruxolitinib or other Ruxolitinib salts, or pharmaceutical formulations of: Ruxolitinib, other Ruxolitinib salts, solid state forms of Ruxolitinib or other Ruxolitinib salts.

## Patentansprüche

1. Salz von Ruxolitinib mit Oxalsäure.

2. Ruxolitinibsalz nach Anspruch 1, **gekennzeichnet durch** ein Röntgenpulverbeugungsmuster mit Peaks bei 5,2, 18,2, 21,0, 22,5 und 25,0 ± 0,2 Grad zwei Theta.

3. Ruxolitinibsalz nach einem der vorhergehenden Ansprüche 1-2, weiterhin **gekennzeichnet durch** einen, zwei, drei, vier, fünf oder sechs zusätzliche Röntgenpulverbeugungsmusterpeaks ausgewählt aus 9,0, 9,6, 13,8, 15,7, 17,7 und 22,9 Grad zwei Theta ± 0,2 Grad zwei Theta.

4. Ruxolitinibsalz nach einem der vorhergehenden Ansprüche 1-3, weiterhin **gekennzeichnet durch** eines oder mehrere der Folgenden: zwei scharfe Endotherme mit Onset-Temperaturen bei 170,2 ± 2°C und 346,4 ± 2°C, ein scharfes Endotherm mit einer Onset-Temperatur von 317,6 ± 2°C sowie zwei breiten Signalen bei 186,5 ± 2°C beziehungsweise 265,0 ± 2°C.

5. Ruxolitinibsalz nach einem der vorhergehenden Ansprüche 1-4, wobei es sich bei der Salzform um ein Monooxalat handelt.

6. Pharmazeutische Zusammensetzung, umfassend das Ruxolitiniboxalat nach einem der vorhergehenden Ansprüche 1-5.

7. Pharmazeutische Formulierung, umfassend Ruxolitiniboxalat nach einem der vorhergehenden Ansprüche 1-5 und mindestens einen pharmazeutisch unbedenklichen Exzipienten.

8. Verwendung von Ruxolitiniboxalat nach einem der vorhergehenden Ansprüche 1-5 bei der Herstellung einer pharmazeutischen Zusammensetzung oder Formulierung.

9. Verfahren zur Herstellung der pharmazeutischen Formulierung nach Anspruch 7, bei dem man Ruxolitiniboxalat nach einem der vorhergehenden Ansprüche 1-5 mit mindestens einem pharmazeutisch unbedenklichen Exzipienten kombiniert.

10. Ruxolitiniboxalat nach einem der vorhergehenden Ansprüche 1-5, pharmazeutische Zusammensetzung nach Anspruch 6 oder pharmazeutische Formulierung nach Anspruch 7 zur Verwendung als Medikament.

11. Ruxolitiniboxalat nach einem der vorhergehenden Ansprüche 1-5, pharmazeutische Zusammensetzung nach Anspruch 6 oder pharmazeutische Formulierung nach Anspruch 7 zur Verwendung bei der Behandlung eines an Krebs, Myelofibrose, Polycythemia vera, essentieller Thrombozythämie, hämatologischen Malignitäten und Psoriasis leidenden Subjekts.

12. Verwendung von Ruxolitiniboxalat nach einem der vorhergehenden Ansprüche 1-5 zur Herstellung von: Ruxolitinib, anderen Ruxolitinibsalzen, Festphasenformen von Ruxolitinib oder anderen Ruxolitinibsalzen oder pharmazeutischen Formulierungen von: Ruxolitinib, anderen Ruxolitinibsalzen, Festphasenformen von Ruxolitinib oder anderen Ruxolitinibsalzen.

## Revendications

1. Sel de Ruxolitinib avec l'acide oxalique.

2. Sel de Ruxolitinib selon la revendication 1, **caractérisé par** un diagramme de diffraction des rayons X sur poudre possédant des pics à 5,2, 18,2, 21,0, 22,5 et 25,0 ± 0,2 degrés deux thêta.

3. Sel de Ruxolitinib selon l'une quelconque des revendications précédentes 1 et 2, **caractérisé en outre par** un, deux, trois, quatre, cinq, ou six pics de PXRD supplémentaires choisis parmi 9,0, 9,6, 13,8, 15,7, 17,7 et 22,9 degrés deux thêta ± 0,2 degrés deux thêta.

4. Sel de Ruxolitinib selon l'une quelconque des revendications précédentes 1 à 3, **caractérisé en outre par** l'un ou plusieurs parmi les suivants : par deux endothermes étroits avec des températures d'initiation à 170,2 ± 2 °C et 346,4 ± 2 °C, un exotherme étroit avec une température d'initiation de 317,6 ± 2 °C, ainsi que deux signaux larges à 186,5 ± 2 °C et 265,0 ± 2 °C, respectivement.

5. Sel de Ruxolitinib selon l'une quelconque des revendications précédentes 1 à 4, la forme saline étant un monooxalate.

6. Composition pharmaceutique comprenant l'oxalate de Ruxolitinib selon l'une quelconque des revendications précédentes 1 à 5.

7. Formulation pharmaceutique comprenant l'oxalate de Ruxolitinib selon l'une quelconque des revendications précédentes 1 à 5, et au moins un excipient pharmaceutiquement acceptable.

8. Utilisation de l'oxalate de Ruxolitinib selon l'une quelconque des revendications précédentes 1 à 5 dans la fabrication d'une composition ou formulation pharmaceutique.

9. Procédé pour la préparation de la formulation pharmaceutique selon la revendication 7, comprenant la combinaison de l'oxalate de Ruxolitinib selon l'une quelconque des revendications précédentes 1 à 5, avec au moins un excipient pharmaceutiquement acceptable.

10. Oxalate de Ruxolitinib à selon l'une quelconque des revendications 1 à 5, composition pharmaceutique selon la revendication 6, ou formulation pharmaceutique selon la revendication 7 pour une utilisation en tant que médicament.

11. Oxalate de Ruxolitinib selon l'une quelconque des revendications précédentes 1 à 5, composition pharmaceutique selon la revendication 6, ou formulation pharmaceutique selon la revendication 7 pour une utilisation dans le traitement d'un sujet souffrant d'un cancer, d'une myélofibrose, de la maladie de Vaquez, d'une thrombocytémie essentielle, de malignités hématologiques et du psoriasis.

12. Utilisation de l'oxalate de Ruxolitinib selon l'une quelconque des revendications précédentes 1 à 5 pour la préparation : de Ruxolitinib, d'autres sels de Ruxolitinib, de formes à l'état solide du Ruxolitinib ou d'autres sels de Ruxolitinib, ou de formulations pharmaceutiques : de Ruxolitinib, d'autres sels de Ruxolitinib, de formes à l'état solide du Ruxolitinib ou d'autres sels de Ruxolitinib.
